# EUROPÄISCHE PATENTSCHRIFT

(11) **EP 2 536 679 B1**
(45) Veröffentlichungstag und Bekanntmachung des Hinweises auf die Patenterteilung: **23.11.2016**
(21) Anmeldenummer: 11700257.6
(22) Anmeldetag: 11.01.2011
(51) Int. Cl.: C07C 49/798, C07C 49/84, C07D 317/54, C11D 3/39, A61K 8/35, A61Q 17/04, A61Q 19/10, A61Q 5/02, C11B 9/00, C11D 3/20, C11D 3/50

(54) **PHOTOLABILE DUFTSPEICHERSTOFFE**
PHOTOLABILE PRO-FRAGRANCES
SUBSTANCES PHOTOLABILES RENFERMANT UN PARFUM

(30) Priorität: 17.02.2010 DE 102010002006
(43) Veröffentlichungstag der Anmeldung: 26.12.2012
(73) Patentinhaber: Henkel AG & Co. KGaA, 40589 Düsseldorf (DE)
(72) Erfinder: GERKE, Thomas, 40627 Düsseldorf (DE); KROPF, Christian, 40724 Hilden (DE); HUCHEL, Ursula, 50670 Köln (DE); GRIESBECK, Axel, 50937 Köln (DE); HINZE, Olga, 50679 Köln (DE)
(86) Internationale Anmeldenummer: PCT/EP2011/050243
(87) Internationale Veröffentlichungsnummer: WO 2011/101180

(56) Entgegenhaltungen:
- WO-A1-2009/118219
- SU-A1- 717 031
- US-A1- 2007 264 217
- US-B2- 6 949 680
- SHU KOBAYASHI ET AL: "NOVEL REACTIVE SILYL ENOLATES. HIGHLY STEREOSELECTIVE ALDOL AND MICHAEL REACTIONS WITHOUT CATALYSTS", JOURNAL OF ORGANIC CHEMISTRY, AMERICAN CHEMICAL SOCIETY, EASTON.; US, Bd. 58, Nr. 10, 7. Mai 1993 (1993-05-07), Seiten 2647-2649, XP000360875, ISSN: 0022-3263, DOI: DOI:10.1021/JO00062A002
- C DELL'ERBA: "[alpha]-arylation vs. [alpha]-arylhydrazonylation of alkyl aryl ketones with arylazo tert-butyl sulfides", TETRAHEDRON, Bd. 49, Nr. 1, 1. Januar 1993 (1993-01-01) , Seiten 235-242, XP55003234, ISSN: 0040-4020, DOI: 10.1016/S0040-4020(01)80522-6
- CLAYTON H. HEATHCOCK ET AL: "Acyclic stereoselection. 29. Stereoselection in the Michael addition reaction. 1. The Mukaiyama-Michael reaction", JOURNAL OF THE AMERICAN CHEMICAL SOCIETY, Bd. 107, Nr. 9, 1. Mai 1985 (1985-05-01), Seiten 2797-2799, XP55003240, ISSN: 0002-7863, DOI: 10.1021/ja00295a037
- DAVID BENSA ET AL: "P-BEMP: A New Efficient and Commercially Available User-Friendly and Recyclable Heterogeneous Organocatalyst for the Michael Addition of 1,3-Dicarbonyl Compounds", SYNTHESIS, Nr. 6, 1. Januar 2004 (2004-01-01), Seiten 923-927, XP55003248, ISSN: 0039-7881, DOI: 10.1055/s-2004-815996
- ZHIYONG JIANG ET AL: "Synthesis of [alpha]-Stereogenic Amides and Ketones by Enantioselective Conjugate Addition of 1,4-Dicarbonyl But-2-enes", CHEMISTRY - A EUROPEAN JOURNAL, Bd. 15, Nr. 19, 4. Mai 2009 (2009-05-04), Seiten 4925-4930, XP55003246, ISSN: 0947-6539, DOI: 10.1002/chem.200802601

## Beschreibung

Die vorliegende Erfindung liegt auf dem Gebiet der Duftspeicherstoffe, wie sie zum Beispiel auf dem Gebiet der Wasch- oder Reinigungsmittel, kosmetischen Mittel sowie Luftpflegemittel Einsatz finden. Die Erfindung betrifft insbesondere spezielle Ketone, die als photolabile Duftspeicherstoffe fungieren. Ferner betrifft die vorliegende Erfindung Wasch- oder Reinigungsmittel, kosmetische Mittel sowie Luftpflegemittel, welche solche Ketone enthalten. Ferner betrifft sie ein Verfahren zur lang anhaltenden Beduftung von Oberflächen und ebenso ein Verfahren zur lang anhaltenden Raumbeduftung.

Wasch- oder Reinigungsmittel bzw. kosmetische Mittel enthalten zumeist Duftstoffe, die den Mitteln einen angenehmen Geruch verleihen. Die Duftstoffe maskieren dabei zumeist den Geruch der anderen Inhaltstoffe, so dass beim Verbraucher ein angenehmer Geruchseindruck entsteht.

Insbesondere im Bereich Waschmittel sind Duftstoffe wichtige Bestandteile der Zusammensetzung, da die Wäsche sowohl im feuchten als auch im trockenen Zustand einen angenehmen und möglichst auch frischen Duft aufweisen soll. Man steht beim Einsatz von Duftstoffen grundsätzlich vor dem Problem, dass es sich bei diesen um mehr oder minder leicht flüchtige Verbindungen handelt, aber dennoch ein lange anhaltender Dufteffekt angestrebt wird. Insbesondere bei denjenigen Riechstoffen, die die frischen und leichten Noten des Parfüms darstellen und infolge ihres hohen Dampfdruck besonders schnell abdampfen, ist die gewünschte Langlebigkeit des Dufteindrucks kaum erreichbar.

Eine verzögerte Duftfreisetzung kann z.B. durch Träger-gebundenen Einsatz von Duftstoffen erfolgen. Eine Träger-gebundene Vorform eines Duftstoffes wird auch als "Pro-Fragrance" oder Duftspeicherstoff bezeichnet. In diesem Zusammenhang offenbart die internationale Patentanmeldung WO2007/087977 die Verwendung von 1-Aza-3,7-dioxabicyclo[3.3.0]octan-Verbindungen als Duftspeicherstoffe zur verzögerten Freisetzung von Duftaldehyden und Duftketonen durch Hydrolyse. Eine alternative Möglichkeit der verzögerten Freisetzung von Duftstoffen stellt der Einsatz von so genannten photoaktivierbaren Substanzen als Duftspeicherstoffe dar. Durch die Einwirkung des Sonnenlichts oder einer anderen elektromagnetischen Strahlungsquelle bestimmter Wellenlänge wird der Bruch einer kovalenten Bindung im Duftspeicherstoff-Molekül induziert, wodurch ein Duftstoff freigesetzt wird.

Das US-Patent 6,949,680 offenbart die Verwendung bestimmter Phenyl- oder Pyridylketone als photoaktivierbare Substanzen, die in Gegenwart von Licht in einer photochemischen Fragmentierung ein terminales Alken als Aktivstoff freisetzen. Der genannte Aktivstoff besitzt beispielsweise eine duftgebende oder antimikrobielle Aktivität, die durch den photochemisch induzierten Zerfall erst verzögert und über einen längeren Zeitraum hinweg auf einer bestimmten Oberfläche freigesetzt wird. WO 2009/118219 A1 offenbart photoaktivierbare Substanzen, welche eine Freisetzung von cyclischen Terpenen oder cyclischen Terpenoiden ermöglichen.

US 2007/0264217 A1 offenbart photoaktivierbare Substanzen, die nach Isomerisierung einen Duftspeicherstoff freisetzen. Die so erhaltenen Duftspeicherstoffe können in einem nachgelagerten Schritt durch einen erneute Fragmentierung einen Riechstoff, insbesondere Duftketone, freisetzen.

Kobayashi et. Al. (J. Org. Chem. 1993, 58, 2647-2649) offenbaren neuartige reaktive Silylenolate, die ohne Katalysator hoch stereoselektive Aldol und Michael-Reaktionen eingehen.

Dell'Erba et. Al. (Tetrahedron 1993, 49(1), 235-242) offenbaren Studien zur alpha-Arylierung bzw. alpha-Arylhydrazonylierung von Alkylarylketonen mit Arylazo-tert-butyl-sulfiden.

Heathcock et. Al. (J. Am. Chem. Soc. 1985, 107, 2797-2799) offenbaren stereoselektive Michael-Additions-Reaktionen, die als Mokaiyama-Michael-Reaktionen bezeichnet werden.

Bensa et. Al. (Synthesis 2004, 6, 923-927) offenbaren neue effiziente und kommerziell erhältliche benutzerfreundliche und wiederverwertbare heterogene Organokatalysatoren für die Michael-Addition von 1,3-Dicarbonyl-Verbindungen.

Jiang et. Al. (Chem. Eur. J. 2009, 15 ,4925-4930) offenbaren die Synthese von alpha-stereogenen Amiden und Ketonen durch enantioselektive Konjugat-Addition von 1,4-Dicarbonylbut-2-enen.

Aufgabe der vorliegenden Erfindung war die Bereitstellung photoaktivierbarer Substanzen als Duftspeicherstoffe, welche die verzögerte Freisetzung von Riechstoffketonen, insbesondere von Damascone erlauben.

Gelöst wurde diese Aufgabe durch ein Keton der allgemeinen Formel (I), 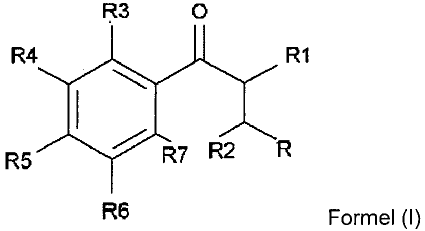 wobei in dieser Formel (I)
R für einen Kohlenwasserstoffrest steht, der mindestens eine C=O-Gruppe aufweist,
R1 für eine lineare oder verzweigte Alkylgruppe mit bis zu 6 C-Atomen,
R2, R3, R4, R5, R6 und R7 unabhängig voneinander für Wasserstoff, ein Halogenatom, NO₂, eine lineare oder verzweigte Alkoxygruppe mit bis zu 15 C-Atomen oder eine lineare oder verzweigte Alkylgruppe mit bis zu 15 C-Atomen stehen, wobei die Reste R und R2 so gewählt sind, dass das Keton der allgemeinen Formel (I) ein Duftstoffspeicher für Duftketone ausgewählt aus Buccoxim, iso-Jasmon, Methyl-beta-naphthylketon, Moschusindanon, Tonalid/Musk plus, alpha-Damascon, beta-Damascon, delta-Damascon, gamma-Damascon, Damascenon, Damarose, Methyldihydrojasmonat, Menthon, Carvon, Kampfer, Fenchon, alpha-Ionon, beta-Ionon, gamma-Methyl genannt Ionon, Fleuramon, Dihydrojasmon, cis-Jasmon, Methylcedrenylketon oder Methylcedrylon, Acetophenon, Methylacetophenon, para-Methoxyacetophenon, Methyl-beta-naphtylketon, Benzylaceton, Benzophenon, para-Hydroxyphenylbutanon, Sellerie-Keton oder Livescone, 6-Isopropyldecahydro-2-naphton, Dimethyloctenon, Frescomenthe, 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)-yl)-1-propanon, 4-(4-Hydroxy-3-methoxyphenyl)-2-butanon, 2-Acetyl-3,3-dimethylnorbornan, 6,7-Dihydro-1,1,2,3,3-pentamethyl-4(5H)-indanon, 4-Damascol, Dulcinyl oder Cassion, Gelson, Hexalon, Isocyclemon E, Methylcyclocitron, Methyllavendelketon, Orivon, para-tertiärem Butylcyclohexanon, Verdon, Delphon, Muscon, Neobutenon, Plicaton, Velouton, 2,4,4,7-Tetramethyl-oct-6-en-3-on, Tetrameran oder Mischungen davon ist.

Es konnte überraschend gefunden werden, dass die erfindungsgemäßen Ketone besonders wirksame Duftspeicherstoffe sind, welche die verzögerte Freisetzung von Riechstoffketonen, insbesondere von Damascone erlauben. Die Anwendung der erfindungsgemäßen Ketone in Wasch-, Reinigungs- oder Pflegemitteln führte bei deren Anwendung zu einer verbesserte Langzeitduftwirkung, insbesondere im Zusammenhang mit der Textilbehandlung. Z.B. konnte bei der Anwendung erfindungsgemäßer Ketone in einem Wäschebehandlungsmittels, wie z.B. Waschmittel sowie Weichspüler, eine verbesserte Langzeitduftwirkung der behandelten Wäsche gefunden werden. Ferner weisen entsprechende Produkte eine besonders gute Lagerstabilität auf. Die erfindungsgemäßen Mittel ermöglichen es zudem, die Gesamtmenge an Parfüm, welche im Mittel enthalten
ist, zu reduzieren, und dennoch Geruchsvorteile auf den gewaschenen Textilien zu erzielen, insbesondere mit Blick auf das Frischeempfinden.

Das erfindungsgemäße Keton gemäß der allgemeinen Formel (I) ist als Duftspeicherstoff für alle üblichen Duftketone ausgewählt aus Buccoxim, iso-Jasmon, Methyl-beta-naphthylketon, Moschusindanon, Tonalid/Musk plus, alpha-Damascon, beta-Damascon, delta-Damascon, gamma-Damascon, Damascenon, Damarose, Methyldihydrojasmonat, Menthon, Carvon, Kampfer, Fenchon, alpha-Ionon, beta-Ionon, gamma-Methyl genannt Ionon, Fleuramon, Dihydrojasmon, cis-Jasmon, Methylcedrenylketon oder Methylcedrylon, Acetophenon, Methylacetophenon, para-Methoxyacetophenon, Methyl-beta-naphtylketon, Benzylaceton, Benzophenon, para-Hydroxyphenylbutanon, Sellerie-Keton oder Livescone, 6-Isopropyldecahydro-2-naphton, Dimethyloctenon, Frescomenthe, 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)-yl)-1-propanon, 4-(4-Hydroxy-3-methoxyphenyl)-2-butanon, 2-Acetyl-3,3-dimethylnorbornan, 6,7-Dihydro-1,1,2,3,3-pentamethyl-4(5H)-indanon, 4-Damascol, Dulcinyl oder Cassion, Gelson, Hexalon, Isocyclemon E, Methylcyclocitron, Methyllavendelketon, Orivon, para-tertiärem Butylcyclohexanon, Verdon, Delphon, Muscon, Neobutenon, Plicaton, Velouton, 2,4,4,7-Tetramethyl-oct-6-en-3-on, Tetrameran oder Mischungen davon geeignet. Bevorzugt können die Ketone ausgewählt sein aus den Damasconen, Carvon, Gamma-Methyl-ionon, Iso-E-Super, 2,4,4,7-Tetramethyl-oct-6-en-3-on, Benzylaceton, Damascenon, Methyldihydrojasmonat, Methylcedrylon, Hedion und Gemischen davon. Am meisten bevorzugt sind alle Damascone sowie Damascenone. Durch Einwirkung von Licht umfassend die Wellenlängen von 200 bis 400 nm können die gespeicherten Ketone freigesetzt werden.

Gemäß einer bevorzugten Ausführungsform der Erfindung steht der Substituent R2 in der Formel (I) für eine lineare oder verzweigte Alkylgruppe mit bis zu 6 C-Atomen, vorzugsweise 1 bis 3 C-Atomen, insbesondere handelt es sich um einen Methylrest.

Gemäß einer weiteren bevorzugten Ausführungsform der Erfindung stehen die Substituenten R3, R4, R5, R6 und R7 in der Formel (I) für Wasserstoff.

Weiterhin ist ein erfindungsgemäßes Keton der allgemeinen Formel (I) bevorzugt, in dem vier der fünf Arylsubstituenten R3, R4, R5, R6 und R7 für Wasserstoff stehen. Vorzugsweise stehen R3, R4, R6 und R7 für Wasserstoff, während der Substituent R5 für ein Halogenatom, insbesondere -F, -Cl oder -Br, NO₂, eine lineare oder verzweigte Alkoxygruppe mit bis zu 6 C-Atomen oder eine lineare oder verzweigte Alkylgruppe mit bis zu 6 C-Atomen steht. In einer weiter bevorzugten Ausführungsform der Erfindung steht R5 in der Formel (I) für -Cl, -Br, -NO₂ oder eine Alkyl- oder Alkoxygruppe umfassend 1 bis 4 C-Atome. Vorzugsweise handelt es sich bei R5 um eine Methyl- oder Ethylgruppe oder um eine Methoxy-,

Ethoxy-, Isopropoxy- oder tert-Butoxy-Gruppe. Eine Substitution in para-Position (R5) ist besonders bevorzugt, da die elektronische Struktur des aromatischen Rings hier am effektivsten modifiziert werden kann, wodurch das Absorptionsmaximum von Ketonen der allgemeinen Formel (I) leicht an eine bestimmte Wellenlänge angepasst werden kann.

Ganz besonders bevorzugt ist es, wenn in der Formel (I) die Substituenten R3, R6, R7 für Wasserstoff stehen, der Rest R4 für Wasserstoff oder eine Alkoxygruppe, insbesondere eine Methoxygruppe, und der Rest R5 für eine Alkoxygruppe, insbesondere eine Methoxygruppe steht.

Gemäß einer bevorzugten Ausführungsform der Erfindung sind Ketone entsprechend den nachfolgenden Formeln (II), (IV) oder Formel (V) besonders bevorzugt: 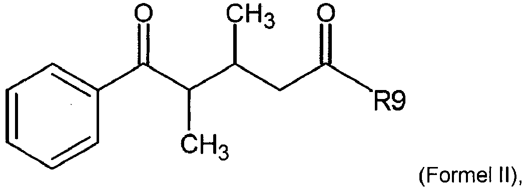 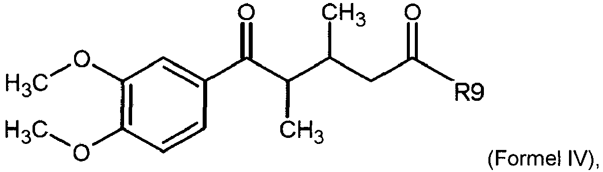 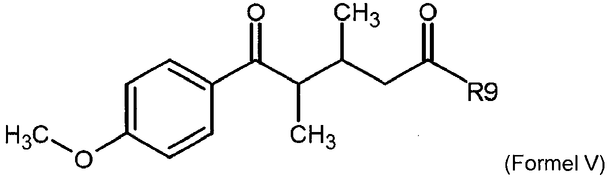 wobei in diesen Formeln (II), (IV) und (V) der Rest R9 für einen Kohlenwasserstoffrest mit wenigstens 5 C-Atomen steht, der insbesondere einen cyclischen Kohlenwasserstoffrest umfasst.

Gemäß einer weiteren bevorzugten Ausführungsform der Erfindung sind Ketone entsprechend den Formeln (VI) bis (X) und (XVI) bis (XXV) bevorzugt:

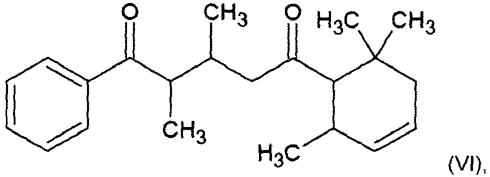

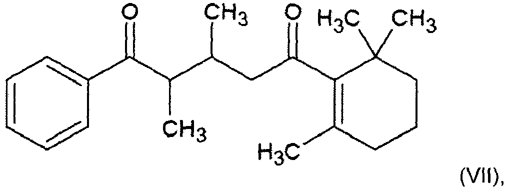

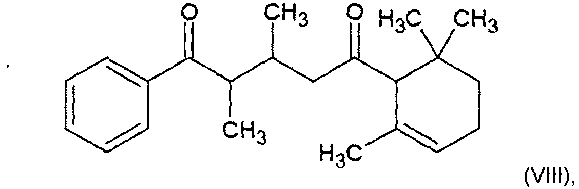

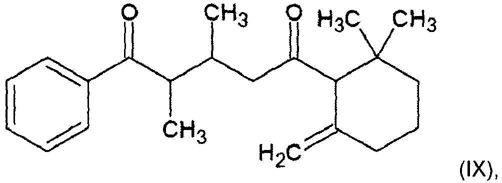

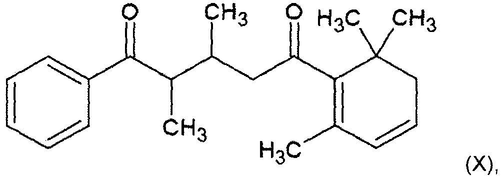

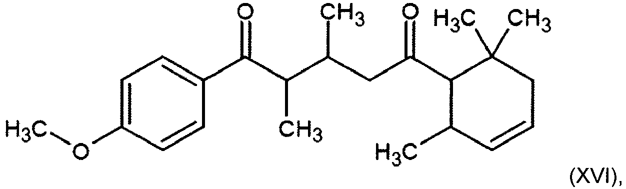

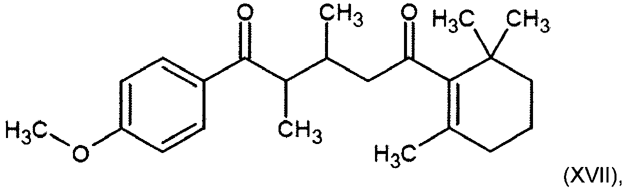

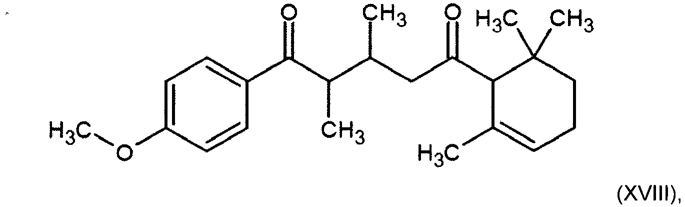

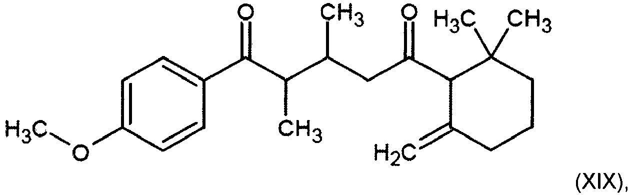

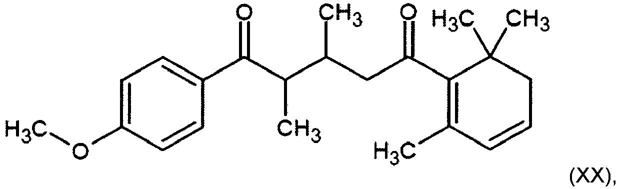

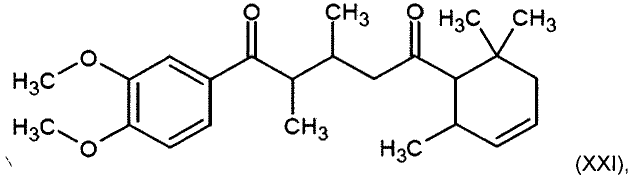

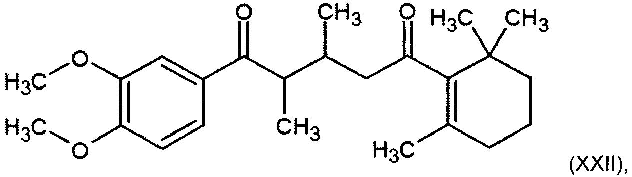

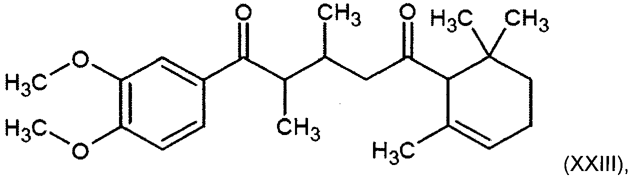

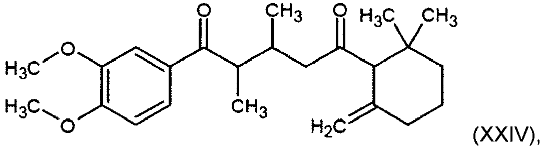

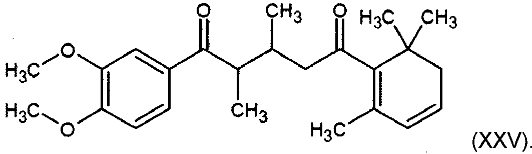

Diese Ketone der vorgenannten Formeln (VI) bis (X) und (XVI) bis (XXV) lassen sich stabil in die üblichen Wasch- oder Reinigungsmittelmatrices, in Kosmetika und bestehende Riechstoffkompositionen einarbeiten. Sie ermöglichen eine verzögerte Freisetzung der gespeicherten Duftstoffe, nämlich von Damascone in der α-, β-, γ- oder Δ-Form sowie von Damascenone, insbesondere β-Damascenone. Diese Ketone verleihen üblichen Wasch- oder Reinigungsmitteln sowie Kosmetika einen besonders lange anhaltenden Frischeeindruck. Insbesondere das getrocknete, gewaschene Textil profitiert von der guten Langzeitfrischeduftwirkung. Die langsame Freisetzung des gespeicherten Riechstoffes erfolgt nach Einwirkung von Licht (elektromagnetische Strahlung) umfassend die Wellenlängen von 200 bis 400 nm, wie in der nachfolgender Reaktionsgleichung vereinfacht veranschaulicht:

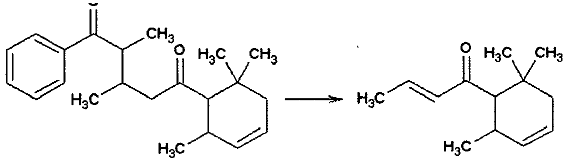

Ein weiterer Gegenstand der vorliegenden Erfindung ist ein Wasch- oder Reinigungsmittel, vorzugsweise ein Waschmittel, Weichspüler oder Waschhilfsmittel, enthaltend mindestens ein Keton gemäß einer der Formeln (I) bis (XXV), wobei das genannte Keton vorzugsweise in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,1 und 2 Gew.-%, jeweils bezogen auf das gesamte Mittel, enthalten ist. Geeignete Reinigungsmittel sind z.B. Reinigungsmittel für harte Oberflächen, wie vorzugsweise Geschirrspülmittel. Ebenso kann es sich um Reinigungsmittel wie z.B. Haushaltsreiniger, Allzweckreiniger, Fensterreiniger, Fußbodenreiniger usw. handeln. Vorzugsweise kann es sich um ein Produkt zur Reinigung von WC-Becken und Urinalen handeln, vorteilhafterweise um einen Spülreiniger zum Einhängen in das WC-Becken, insbesondere um einen sogenannten WC-Stein.

Gemäß einer bevorzugten Ausführungsform der Erfindung enthält das erfindungsgemäße Wasch- oder Reinigungsmittel mindestens ein Tensid, ausgewählt aus anionischen, kationischen, nichtionischen, zwitterionischen, amphoteren Tensiden oder Mischungen daraus.

Gemäß einer weiteren bevorzugten Ausführungsform der Erfindung liegt das erfindungsgemäße Mittel in fester oder flüssiger Form vor.

Eine weitere Ausführungsform der Erfindung ist ein kosmetisches Mittel, enthaltend mindestens ein Keton gemäß einer der Formeln (I) bis (XXV), welches das genannte Keton vorzugsweise in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,1 und 2 Gew.-%, jeweils bezogen auf das gesamte Mittel, enthält.

Eine weitere Ausführungsform der Erfindung ist ein Luftpflegemittel (z.B. Raumlufterfrischer, Raumdeodorant, Raumspray usw.), enthaltend mindestens ein Keton gemäß einer der Formeln (I) bis (XXV), wobei das genannte Keton vorzugsweise in Mengen zwischen 0,0001 und 50 Gew.-%, vorteilhafterweise zwischen 0,001 und 5 Gew.-%, weiter vorteilhaft zwischen 0,1 und 3 Gew.-%, insbesondere zwischen 0,1 und 2 Gew.-%, jeweils bezogen auf das gesamte Mittel, enthalten ist.

Nach einer weiteren bevorzugten Ausführungsform der Erfindung sind in einem erfindungsgemäßen Mittel (d.h. Wasch- oder Reinigungsmittel, kosmetisches Mittel, oder Luftpflegemittel) zusätzliche Duftstoffe enthalten, insbesondere ausgewählt aus der Gruppe umfassend Duftstoffe natürlichen oder synthetischen Ursprungs, bevorzugt leichter flüchtige Duftstoffe, höhersiedende Duftstoffe, feste Duftstoffe und/oder haftfeste Duftstoffe.

Haftfeste Riechstoffe, die im Rahmen der vorliegenden Erfindung mit Vorteil einsetzbar sind, sind beispielsweise etherische Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl,

Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.

Aber auch höhersiedende bzw. feste Riechstoffe natürlichen oder synthetischen Ursprungs können im Rahmen der vorliegenden Erfindung als haftfeste Riechstoffe bzw. Riechstoffgemische, also Duftstoffe eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylalkohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresol-methylether, Cumarin, p-Methoxyacetophenon, Methyl-n-amylketon, Methylanthranil-säure-methylester, p-Methylacetophenon, Methylchavikol, p-Methyl-chinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadekanolid, β-Phenylethyl-alkohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanillin, Veratrumaldehyd, Zimtaldehyd, Zimatalkohol, Zimtsäure, Zimtsäureethylester, Zimtsäurebenzylester. Zu den leichter flüchtigen Duftstoffen zählen insbesondere die niedriger siedenden Riechstoffe natürlichen oder synthetischen Ursprung, die allein oder in Mischungen eingesetzt werden können. Beispiele für leichter flüchtige Duftstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral, Zitronellal.

Nach einer weiteren bevorzugten Ausführungsform weist das erfindungsgemäße Mittel (d.h. Wasch- oder Reinigungsmittel, kosmetisches Mittel, oder Luftpflegemittel), wenigstens eine, vorzugsweise mehrere, aktive Komponenten, insbesondere wasch-, pflege-, reinigungsaktive und/oder kosmetische Komponenten auf, vorteilhafterweise ausgewählt aus der Gruppe umfassend anionische Tenside, kationische Tenside, amphotere Tenside, nichtionische Tenside, Acidifizierungsmittel, Alkalisierungsmittel, Anti-Knitter-Verbindungen, antibakterielle Stoffe, Antioxidantien, Antiredepositionsmittel, Antistatika, Buildersubstanzen, Bleichmittel, Bleichaktivatoren, Bleichstabilisatoren, Bleichkatalysatoren, Bügelhilfsmittel, Cobuilder, Duftstoffe, Einlaufverhinderer, Elektrolyte, Enzyme, Farbschutzstoffe, Färbemittel, Farbstoffe, Farbübertragungsinhibitoren, Fluoreszenzmittel, Fungizide, Germizide, geruchskomplexierende Substanzen, Hilfsmittel, Hydrotrope, Klarspüler, Komplexbildner, Konservierungsmittel, Korrosionsinhibitoren, wassermischbare organische Lösungsmittel, optische Aufheller, Parfüme, Parfümträger, Perlglanzgeber, pH-Stellmittel, Phobier- und Imprägniermittel, Polymere, Quell- und Schiebefestmittel, Schauminhibitoren, Schichtsilikate, schmutzabweisende Stoffe, Silberschutzmittel, Silikonöle, Soilrelease-Wirkstoffe, UV-Schutz-Substanzen, Viskositätsregulatoren, Verdickungsmittel, Verfärbungsinhibitoren, Vergrauungsinhibitoren, Vitamine und/oder Weichspüler. Im Sinne dieser Erfindung beziehen sich Angaben für das erfindungsgemäße Mittel in Gew.-%, wenn nicht anders angegeben, auf das Gesamtgewicht des erfindungsgemäßen Mittels.

Die Mengen der einzelnen Inhaltsstoffe in den erfindungsgemäßen Mitteln (d.h. Wasch- oder Reinigungsmittel, kosmetisches Mittel oder Luftpflegemittel) orientieren sich jeweils am Einsatzzweck der betreffenden Mittel und der Fachmann ist mit den Größenordnungen der einzusetzenden Mengen der Inhaltsstoffe grundsätzlich vertraut oder kann diese der zugehörigen Fachliteratur entnehmen. Je nach Einsatzzweck der erfindungsgemäßen Mittel wird man beispielsweise den Tensidgehalt höher oder niedriger wählen. Üblicherweise kann z. B. der Tensidgehalt beispielsweise von Waschmitteln zwischen 10 und 50 Gew.-%, vorzugsweise zwischen 12,5 und 30 Gew.-% und insbesondere zwischen 15 und 25 Gew.-% betragen, während z.B. Reinigungsmittel für das maschinelle Geschirrspülen z.B. zwischen 0,1 und 10 Gew.-%, vorzugsweise zwischen 0,5 und 7,5 Gew.-% und insbesondere zwischen 1 und 5 Gew.-% Tenside enthalten können.

Die erfindungsgemäßen Mittel (d.h. Wasch- oder Reinigungsmittel, kosmetisches Mittel oder Luftpflegemittel) können Tenside enthalten, wobei bevorzugt anionische Tenside, nichtionische Tenside und deren Gemische, aber auch kationische Tenside in Frage kommen. Geeignete nichtionische Tenside sind insbesondere Ethoxylierungs- und/oder Propoxylierungsprodukte von Alkylglykosiden und/oder linearen oder verzweigten Alkoholen mit jeweils 12 bis 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Geeignete anionische Tenside sind insbesondere Seifen und solche, die Sulfat- oder SulfonatGruppen mit bevorzugt Alkaliionen als Kationen enthalten. Verwendbare Seifen sind bevorzugt die Alkalisalze der gesättigten oder ungesättigten Fettsäuren mit 12 bis 18 C-Atomen. Derartige Fettsäuren können auch in nicht vollständig neutralisierter Form eingesetzt werden. Zu den brauchbaren Tensiden des Sulfat-Typs gehören die Salze der Schwefelsäurehalbester von Fettalkoholen mit 12 bis 18 C-Atomen und die Sulfatierungsprodukte der genannten nichtionischen Tenside mit niedrigem Ethoxylierungsgrad. Zu den verwendbaren Tensiden vom Sulfonat-Typ gehören lineare Alkylbenzolsulfonate mit 9 bis 14 C-Atomen im Alkylteil, Alkansulfonate mit 12 bis 18 C-Atomen,
sowie Olefinsulfonate mit 12 bis 18 C-Atomen, die bei der Umsetzung entsprechender Monoolefine mit Schwefeltrioxid entstehen, sowie alpha-Sulfofettsäureester, die bei der Sulfonierung von Fettsäuremethyl- oder -ethylestern entstehen.

Kationische Tenside werden vorzugsweise unter den Esterquats und/oder den quaternären Ammoniumverbindungen (QAV) gemäß der allgemeinen Formel (R^{I})(R^{II})(R^{III})(R^{IV})N⁺ X- ausgewählt, in der R^{I} bis R^{IV} für gleiche oder verschiedene C₁₋₂₂-Alkylreste, C₇₋₂₈-Arylalkylreste oder heterozyklische Reste stehen, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, und X- für Halogenidionen, Sulfationen, Hydroxidionen oder ähnliche Anionen steht. QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer Methyl-Gruppe kann mit Hilfe von Methylchlorid unter milden Bedingungen durchgeführt werden. Amine, die über drei lange Alkyl-Reste oder Hydroxysubstituierte Alkyl-Reste verfügen, sind wenig reaktiv und werden z.B. mit Dimethylsulfat quaterniert. In Frage kommende QAV sind beispielweise Benzalkoniumchlorid (N Alkyl-N,N dimethyl-benzylammoniumchlorid), Benzalkon B (m,p-Dichlorbenzyldimethyl-C₁₂-alkylammoniumchlorid, Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid), Benzetoniumchlorid (N,N Dimethyl-N [2-[2-[p-(1,1,3,3-tetramethylbutyl)phenoxy]-ethoxy]-ethyl]-benzyl-ammoniumchlorid), Dialkyldimethylammoniumchloride wie Di-n-decyl-dimethyl-ammoniumchlorid, Didecyldimethylammonium-bromid, Dioctyl-dimethyl-ammoniumchlorid, 1-Cetylpyridiniumchlorid und Thiazoliniodid sowie deren Mischungen. Bevorzugte QAV sind die Benzalkoniumchloride mit C₈-C₂₂-Alkylresten, insbesondere C₁₂-C₁₄-Alkyl-benzyldimethylammoniumchlorid.
Bevorzugte Esterquats sind Methyl-N-(2-hydroxyethyl)-N,N-di(talgacyl-oxyethyl)ammonium-methosulfat, Bis-(palmitoyl)-ethyl-hydroxyethyl-methyl-ammonium-methosulfat oder Methyl-N,N-bis(acyloxyethyl)-N-(2-hydroxyethyl)ammonium-methosulfat. Handelsübliche Beispiele sind die von der Firma Stepan unter dem Warenzeichen Stepantex^{®} vertriebenen Methylhydroxyalkyldialkoyloxyalkylammoniummethosulfate oder die unter dem Handelsnamen Dehyquart^{®} bekannten Produkte der Firma Cognis Deutschland GmbH beziehungsweise die unter der Bezeichnung Rewoquat^{®} bekannten Produkte des Herstellers Goldschmidt-Witco.

Tenside sind in den erfindungsgemäßen Mitteln (d.h. Wasch- oder Reinigungsmittel, kosmetisches Mittel, oder Luftpflegemittel) in Mengenanteilen von vorzugsweise 5 Gew.-% bis 50 Gew.-%, insbesondere von 8 Gew.-% bis 30 Gew.-%, enthalten. Insbesondere in Wäschenachbehandlungsmitteln werden vorzugsweise bis zu 30 Gew.-%, insbesondere 5 Gew.-% bis 15 Gew.-% Tenside, unter diesen bevorzugt wenigstens anteilsweise Kationtenside, eingesetzt.

Ein erfindungsgemäßes Mittel, insbesondere Wasch- oder Reinigungsmittel, enthält vorzugsweise mindestens einen wasserlöslichen und/oder wasserunlöslichen, organischen und/oder anorganischen Builder. Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Die organischen Buildersubstanzen können, insbesondere zur Herstellung flüssiger Mittel, in Form wäßriger Lösungen, vorzugsweise in Form 30- bis 50-gewichtsprozentiger wäßriger Lösungen eingesetzt werden. Alle genannten Säuren werden in der Regel in Form ihrer wasserlöslichen Salze, insbesondere ihre Alkalisalze, eingesetzt.

Organische Buildersubstanzen können gewünschtenfalls in Mengen bis zu 40 Gew.-%, insbesondere bis zu 25 Gew.-% und vorzugsweise von 1 Gew.-% bis 8 Gew.-% enthalten sein. Mengen nahe der genannten Obergrenze werden vorzugsweise in pastenförmigen oder flüssigen, insbesondere wasserhaltigen, erfindungsgemäßen Mitteln eingesetzt. Erfindungsgemäße Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und Polyphosphate, vorzugsweise Natriumtriphosphat, in Betracht. Als wasserunlösliche, wasserdispergierbare anorganische Buildermaterialien können insbesondere kristalline oder amorphe Alkalialumosilikate, gewünschtenfalls in Mengen von bis zu 50 Gew.-%, vorzugsweise nicht über 40 Gew.-% und in flüssigen Mitteln insbesondere von 1 Gew.-% bis 5 Gew.-%, eingesetzt werden. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂x₊₁ · y H₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl ß- als auch δ-Natriumdisilikate (Na₂Si₂O₅ · y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in erfindungsgemäßen Mitteln eingesetzt werden. In einer weiteren bevorzugten Ausführungsform erfindungsgemäßer Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren bevorzugten Ausführungsform erfindungsgemäßer Mittel eingesetzt. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind gewünschtenfalls in den erfindungsgemäßen Mitteln vorzugsweise in Mengen bis zu 60 Gew.-%, insbesondere von 5 Gew.-% bis 40 Gew.-%, enthalten. Erfindungsgemäße Wäschenachbehandlungsmittel, wie z.B. Weichspüler, sind vorzugsweise frei von anorganischem Builder.

Als geeignete Persauerstoffverbindungen kommen insbesondere organische Persäuren beziehungsweise persaure Salze organischer Säuren, wie Phthalimidopercapronsäure, Perbenzoesäure oder Salze der Diperdodecandisäure, Wasserstoffperoxid und unter den Anwendungsbedingungen Wasserstoffperoxid abgebende anorganische Salze, wie Perborat, Percarbonat und/oder Persilikat, in Betracht. Sofern feste Persauerstoffverbindungen eingesetzt werden sollen, können diese in Form von Pulvern oder Granulaten verwendet werden, die auch in im Prinzip bekannter Weise umhüllt sein können. Besonders bevorzugt wird Alkalipercarbonat, Alkaliperborat-Monohydrat oder insbesondere in flüssigen Mitteln Wasserstoffperoxid in Form wäßriger Lösungen, die 3 Gew.-% bis 10 Gew.-% Wasserstoffperoxid enthalten, ggf. eingesetzt. Falls ein erfindungsgemäßes Mittel Bleichmittel, wie vorzugsweise Persauerstoffverbindungen, enthält, sind diese in Mengen von vorzugsweise bis zu 50 Gew.-%, insbesondere von 5 Gew.-% bis 30 Gew.-%, vorhanden. Der Zusatz geringer Mengen bekannter Bleichmittelstabilisatoren wie beispielsweise von Phosphonaten, Boraten bzw. Metaboraten und Metasilikaten sowie Magnesiumsalzen wie Magnesiumsulfat kann zweckdienlich sein.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxo-hexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und Enolester sowie acetyliertes Sorbitol und Mannitol beziehungsweise deren Mischungen (SORMAN), acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam. Hydrophil substituierte Acylacetale und Acyllactame werden ebenfalls bevorzugt eingesetzt. Auch Kombinationen konventioneller Bleichaktivatoren können eingesetzt werden. Derartige Bleichaktivatoren können im üblichen Mengenbereich, vorzugsweise in Mengen von 1 Gew.-% bis 10 Gew.-%, insbesondere 2 Gew.-% bis 8 Gew.-%, bezogen auf gesamtes Mittel, enthalten sein.

Zusätzlich zu den oben aufgeführten konventionellen Bleichaktivatoren oder an deren Stelle können auch Sulfonimine und/oder bleichverstärkende Übergangsmetallsalze beziehungsweise Übergangsmetallkomplexe als sogenannte Bleichkatalysatoren enthalten sein.

Als in den Mitteln verwendbare Enzyme kommen solche aus der Klasse der Proteasen, Cutinasen, Amylasen, Pullulanasen, Hemicellulasen, Cellulasen, Lipasen, Oxidasen und Peroxidasen sowie deren Gemische in Frage. Besonders geeignet sind aus Pilzen oder Bakterien, wie Bacillus subtilis, Bacillus licheniformis, Streptomyces griseus, Humicola lanuginosa, Humicola insolens, Pseudomonas pseudoalcaligenes oder Pseudomonas cepacia gewonnene enzymatische Wirkstoffe. Die gegebenenfalls verwendeten Enzyme können an Trägerstoffen adsorbiert und/oder in Hüllsubstanzen eingebettet sein, um sie gegen vorzeitige Inaktivierung zu schützen. Sie sind gewünschtenfalls in den erfindungsgemäßen Mitteln vorzugsweise in Mengen nicht über 5 Gew.-%, insbesondere von 0,2 Gew.-% bis 2 Gew.-%, enthalten.
Die Mittel können ggf. als optische Aufheller beispielsweise Derivate der Diaminostilbendisulfonsäure beziehungsweise deren Alkalimetallsalze enthalten. Geeignet sind zum Beispiel Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen.

Zu den geeigneten Schauminhibitoren gehören beispielsweise Organopolysiloxane und deren Gemische mit mikrofeiner, gegebenenfalls silanierter Kieselsäure sowie Paraffinwachse und deren Gemische mit silanierter Kieselsäure oder Bisfettsäure-alkylendiamiden. Mit Vorteilen werden auch Gemische aus verschiedenen Schaum-inhibitoren verwendet, zum Beispiel solche aus Silikonen, Paraffinen oder Wachsen. Vorzugsweise sind die Schauminhibitoren, insbesondere Silikon-und/oder Paraffin-haltige Schauminhibitoren, an eine granulare, in Wasser lösliche beziehungsweise dispergierbare Trägersubstanz gebunden. Insbesondere sind dabei Mischungen aus Paraffinwachsen und Bistearylethylendiamiden bevorzugt.

Zusätzlich können die Mittel auch Komponenten enthalten, welche die Öl- und Fettauswaschbarkeit aus Textilien positiv beeinflussen, sogenannte soil release-Wirkstoffe. Dieser Effekt wird besonders deutlich, wenn ein Textil verschmutzt wird, das bereits vorher mehrfach mit einem erfindungsgemäßen Mittel, das diese öl- und fettlösende Komponente enthält, gewaschen wurde. Zu den bevorzugten öl- und fettlösenden Komponenten zählen beispielsweise nichtionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten mit monomeren und/oder polymeren Diolen, insbesondere Polymere aus Ethylenterephthalaten und/oder Polyethylenglykolterephthalaten oder anionisch und/oder nichtionisch modifizierten Derivaten von diesen.

Die Mittel können auch Farbübertragungsinhibitoren, vorzugsweise in Mengen von 0,1 Gew.-% bis 2 Gew.-%, insbesondere 0,1 Gew.-% bis 1 Gew.-%, enthalten, die in einer bevorzugten Ausgestaltung der Erfindung Polymere aus Vinylpyrrolidon, Vinylimidazol, Vinylpyridin-N-Oxid oder Copolymere aus diesen sind.

Vergrauungsinhibitoren haben die Aufgabe, den von der Textilfaser abgelösten Schmutz in der Flotte suspendiert zu halten. Hierzu sind wasserlösliche Kolloide meist organischer Natur geeignet, beispielsweise Stärke, Leim, Gelatine, Salze von Ethercarbonsäuren oder Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich andere als die obengenannten Stärkederivate verwenden, zum Beispiel Aldehydstärken. Bevorzugt können Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, beispielsweise in Mengen von 0,1 bis 5 Gew.-%, bezogen auf die Mittel, eingesetzt werden.

Zu den in den erfindungsgemäßen Mitteln, insbesondere wenn sie in flüssiger oder pastöser Form vorliegen, verwendbaren organischen Lösungsmitteln gehören Alkohole mit 1 bis 4 C-Atomen, insbesondere Methanol, Ethanol, Isopropanol und tert.-Butanol, Diole mit 2 bis 4 C-Atomen, insbesondere Ethylenglykol und Propylenglykol, sowie deren Gemische und die aus den genannten Verbindungsklassen ableitbaren Ether. Derartige wassermischbare Lösungsmittel sind in den erfindungsgemäßen Mitteln vorzugsweise in Mengen von nicht über 30 Gew.-%, insbesondere von 6 Gew.-% bis 20 Gew.-%, vorhanden.

Zur Einstellung eines gewünschten, sich durch die Mischung der übrigen Komponenten nicht von selbst ergebenden pH-Werts können die erfindungsgemäßen Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure, oder Basen, insbesondere Ammonium- oder Alkalihydroxide, enthalten. Derartige pH-Regulatoren sind optional in den erfindungsgemäßen Mitteln vorzugsweise nicht über 20 Gew.-%, insbesondere von 1,2 Gew.-% bis 17 Gew.-%, enthalten.

Die Herstellung fester erfindungsgemäßer Mittel (d.h. insbesondere Wasch- oder Reinigungsmittel) bereitet keine Schwierigkeiten und kann im Prinzip bekannter Weise, zum Beispiel durch Sprühtrocknen oder Granulation, erfolgen, wobei optionale Persauerstoffverbindung und optionaler Bleichkatalysator gegebenenfalls später zugesetzt werden. Zur Herstellung erfindungsgemäßer Mittel mit erhöhtem Schüttgewicht, insbesondere im Bereich von 650 g/l bis 950 g/l, ist ein einen Extrusionsschritt aufweisendes Verfahren bevorzugt. Die Herstellung flüssiger erfindungsgemäßer Mittel bereitet ebenfalls keine Schwierigkeiten und kann ebenfalls in bekannter Weise erfolgen.

Die Herstellung der erfindungsgemäßen Ketone wird im Beispielteil exemplarisch anhand der Herstellung eines δ-Damascone-enthaltenden Duftspeicherstoffes beschrieben. Über diese prinzipielle Syntheseroute sind auch die anderen Ketone der allgemeinen Formel (I) und insbesondere alle Ketone der Formeln (VI) bis (XXV) zugänglich.

Gemäß einer bevorzugten Ausführungsform kann die erfindungsgemäße Lehre dazu eingesetzt werden, den Parfümanteil in Wasch-, Reinigungs- und Körperpflegemitteln signifikant herabzusetzen. Dadurch ist es möglich, parfümierte Produkte auch für solche besonders empfindlichen Konsumenten anzubieten, die normal parfümierte Produkte aufgrund spezieller Unverträglichkeiten und Irritationen nur eingeschränkt oder gar nicht verwenden können.

Ein bevorzugtes erfindungsgemäßes festes, insbesondere pulverförmiges Waschmittel kann neben dem erfindungsgemäßen Keton insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5-30 Gew.-%
- Nichtionische Tenside, wie vorzugsweise Fettalkoholpolyglycolether, Alkylpolyglucosid, Fettsäureglucamid z.B. in Mengen von vorzugsweise 0,5-15 Gew.-%
- Gerüststoffe, wie z.B. Zeolith, Polycarboxylat, Natriumcitrat, in Mengen von z.B. 0-70 Gew.-% , vorteilhafterweise 5-60 Gew.-%, vorzugsweise 10-55 Gew.-%, insbesondere 15-40 Gew.-%,
- Alkalien, wie z.B. Natriumcarbonat, in Mengen von z.B. 0-35 Gew.-% vorteilhafterweise 1-30 Gew.-%, vorzugsweise 2-25 Gew.-%, insbesondere 5-20 Gew.-%,
- Bleichmittel, wie z.B. Natriumperborat, Natriumpercarbonat, in Mengen von z.B. 0-30 Gew.-% vorteilhafterweise 5-25 Gew.-%, vorzugsweise 10-20 Gew.-%,
- Korrosionsinhibitoren, z.B. Natriumsilicat, in Mengen von z.B. 0-10 Gew.-%, vorteilhafterweise 1-6 Gew.-%, vorzugsweise 2-5 Gew.-%, insbesondere 3-4 Gew.-%,
- Stabilisatoren, z.B. Phosphonate, vorteilhafterweise 0-1 Gew.-%,
- Schauminhibitor, z.B. Seife, Siliconöle, Paraffine vorteilhafterweise 0-4 Gew.-%, vorzugsweise 0,1-3 Gew.-%, insbesondere 0,2-1 Gew.-%,
- Enzyme, z.B. Proteasen, Amylasen, Cellulasen, Lipasen, vorteilhafterweise 0-2 Gew.-%, vorzugsweise 0,2-1 Gew.-%, insbesondere 0,3-0,8 Gew.-%,
- Vergrauungsinhibitor, z.B. Carboxymethylcellulose, vorteilhafterweise 0-1 Gew.-%,
- Verfärbungsinhibitor, z.B. Polyvinylpyrrolidon-Derivate, vorzugsweise 0-2 Gew.-%,
- Stellmittel, z.B. Natriumsulfat, vorteilhafterweise 0-20 Gew.-%,
- Optische Aufheller, z.B. Stilben-Derivat, Biphenyl-Derivat, vorteilhafterweise 0-0,4 Gew.-%, insbesondere 0,1-0,3 Gew.-%,
- ggf. weitere Duftstoffe
- ggf. Wasser
- ggf. Seife
- ggf. Bleichaktivatoren
- ggf. Cellulosederivate
- ggf. Schmutzabweiser,

Gew.-% jeweils bezogen auf das gesamte Mittel.

In einer anderen bevorzugten Ausführungsform der Erfindung liegt das Mittel in flüssiger Form vor, vorzugsweise in Gelform. Bevorzugte flüssige Wasch- oder Reinigungsmittel sowie Kosmetika haben Wassergehalte von z.B. 10-95 Gew.-%, vorzugsweise 20-80 Gew.-% und insbesondere 30-70 Gew.-%, bezogen auf das gesamte Mittel. Im Falle von flüssigen Konzentraten kann der Wassergehalt auch besonders gering sein, z.B. < 30 Gew.-%, vorzugsweise < 20 Gew.-%, insbesondere < 15 Gew.-% betragen, Gew.-% jeweils bezogen auf das gesamte Mittel. Die flüssigen Mittel können auch nichtwässrige Lösungsmittel enthalten.

Ein bevorzugtes erfindungsgemäßes flüssiges, insbesondere gelförmiges Waschmittel kann neben dem erfindungsgemäßen Keton insbesondere noch Komponenten enthalten, die z.B. ausgewählt sind aus den folgenden:
- Aniontenside, wie vorzugsweise Alkylbenzolsulfonat, Alkylsulfat, z.B. in Mengen von vorzugsweise 5-40 Gew.-%
- Nichtionische Tenside, wie vorzugsweise Fettalkoholpolyglycolether, Alkylpolyglucosid, Fettsäureglucamid z.B. in Mengen von vorzugsweise 0,5-25 Gew.-%
- Gerüststoffe, wie z.B. Zeolith, Polycarboxylat, Natriumcitrat, vorteilhafterweise 0-15 Gew.-%, vorzugsweise 0,01-10 Gew.-%, insbesondere 0,1-5 Gew.-%,
- Schauminhibitor, z.B. Seife, Siliconöle, Paraffine, in Mengen von z.B. 0-10 Gew.-%, vorteilhafterweise 0,1-4 Gew.-%, vorzugsweise 0,2-2 Gew.-%, insbesondere 1-3 Gew.-%,
- Enzyme, z.B. Proteasen, Amylasen, Cellulasen, Lipasen, in Mengen von z.B. 0-3 Gew.-%, vorteilhafterweise 0,1-2 Gew.-%, vorzugsweise 0,2-1 Gew.-%, insbesondere 0,3-0,8 Gew.-%,
- Optische Aufheller, z.B. Stilben-Derivat, Biphenyl-Derivat, in Mengen von z.B. 0-1 Gew.-%, vorteilhafterweise 0,1-0,3 Gew.-%, insbesondere 0,1-0,4 Gew.-%,
- ggf. weitere Duftstoffe
- ggf. Stabilisatoren,
- Wasser
- ggf. Seife, in Mengen von z.B. 0-25 Gew.-%, vorteilhafterweise 1-20 Gew.-%, vorzugsweise 2-15 Gew.-%, insbesondere 5-10 Gew.-%,
- ggf. Lösungsmittel (vorzugsweise Alkohole), vorteilhafterweise 0-25 Gew.-%, vorzugsweise 1-20 Gew.-%, insbesondere 2-15 Gew.-%, Gew.-% jeweils bezogen auf das gesamte Mittel.

Ein bevorzugter erfindungsgemäßer flüssiger Weichspüler kann neben dem erfindungsgemäßen Keton insbesondere noch Komponenten enthalten, die ausgewählt sind aus den folgenden:
- Kationische Tenside, wie insbesondere Esterquats, z.B. in Mengen von 5-30 Gew.-%,
- Cotenside, wie z.B. Glycerolmonostearat, Stearinsäure, Fettalkohole, Fettalkoholethoxylate, z.B. in Mengen von 0-5 Gew.-%, vorzugsweise 0,1-4 Gew.-%,
- Emulgatoren, wie z.B. Fettaminethoxylate, z.B. in Mengen von 0-4 Gew.-%, vorzugsweise 0,1-3 Gew.-%,
- ggf. weitere Duftstoffe
- Farbstoffe, vorzugsweise im ppm-Bereich
- Stabilisatoren, vorzugsweise im ppm-Bereich
- Lösemittel, wie z.B. Wasser, in Mengen von vorzugsweise 60-90 Gew.-%,
- Gew.-% jeweils bezogen auf das gesamte Mittel.

Ein weiterer Gegenstand der Erfindung ist ein Verfahren zur lang anhaltenden Beduftung von Oberflächen, wobei ein Keton gemäß einer der Formeln (I) bis (XXV) oder ein erfindungsgemäßes Wasch- oder Reinigungsmittel auf die zu beduftende Oberfläche (z.B. Textil, Geschirr, Fußboden) aufgebracht wird und die genannte Oberfläche anschließend einer elektromagnetischen Strahlung umfassend die Wellenlängen von 200 bis 400 nm ausgesetzt wird.

Ein weiterer Gegenstand der Erfindung ist ein Verfahren zur lang anhaltenden Raumbeduftung, wobei ein erfindungsgemäßes Luftpflegemittel einer elektromagnetischen Strahlung umfassend die Wellenlängen von 200 bis 400 nm ausgesetzt wird.

### Beispiel:

### Darstellung eines Ketons der allgemeinen Formel (I):

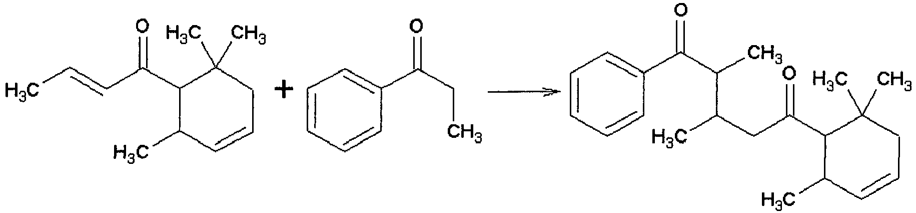

Unter Stickstoff wurden 8,50g Diisopropylamin in 210 ml THF vorgelegt und die Lösung auf -78°C abgekühlt. Danach folgte die Zugabe von 11,3 g Propiophenon und anschliessend die Zugabe von 40,3 ml einer Butyllithium-Lösung (2,5 molar in Hexan; entspricht 100,8 mmol). Die Reaktionslösung ließ man 1 Stunde bei -78°C rühren. Dann erfolgte unter Rühren die Zugabe von 24 g getrocknetem Cerchlorid (Cer-III-chlorid getrocknet; entspricht 98mmol; hergestellt aus: Cer-III-chlorid*7H2O durch sechsstündiges Trocknen bei 150°C im Hochvakuum). Die Reaktionslösung wurde dann 30 Minuten bei -78 °C gerührt. Anschließend wurden 14,8 g δ-Damascone mittels Tropftrichter langsam zugetropft und die Reaktionslösung wurde bei -78 °C weiter gerührt. Danach wurde die Kühlung entfernt und bei einer Temperatur von ca.-10°C erfolgte die Zugabe einer gesättigten Ammoniumchloridlösung. Zur Reinigung wurde 3 Mal mit jeweils 350 ml Ether extrahiert und die resultierende hellgelbe organische Phase wurde danach mit Wasser, später mit gesättigter NaCl-Lösung ausgeschüttelt. Die organische Phase wurde dann über Magnesiumsulfat getrocknet. Das Filtrat wurde bei vermindertem Druck von Lösungsmittel befreit. Das resultierende Rohprodukt wurde schließlich im Hochvakuum destilliert und es resultierte das gewünschte Zielprodukt in einer Menge von 29 g.

Das auf diese Weise hergestellte Keton zeigte bei der Anwendung in Waschmitteln und Weichspülern bei der Textilbehandlung eine sehr gute Duftwirkung. Insbesondere wurde eine bessere Dauerhaftigkeit des Dufteindruckes auf der damit gewaschenen und danach getrockneten Wäsche gefunden, verglichen mit Waschmitteln und Weichspülern die eine äquivalente Menge δ-Damascone enthielten, ansonsten aber gleichgestaltet waren. Der frische Dufteindruck der Textilien hielt deutlich länger vor, sowohl nach Leinentrocknung wie insbesondere nach Trocknung im Maschinentrockner.

## Patentansprüche

1. Keton der allgemeinen Formel (I), 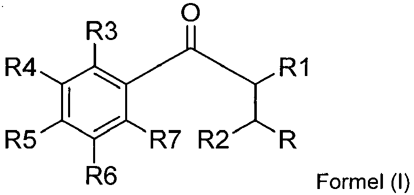 wobei
R für einen Kohlenwasserstoffrest steht, der mindestens eine C=O-Gruppe aufweist,
R1 für eine lineare oder verzweigte Alkylgruppe mit bis zu 6 C-Atomen,
R2, R3, R4, R5, R6 und R7 unabhängig voneinander für Wasserstoff, ein Halogenatom, NO₂, eine lineare oder verzweigte Alkoxygruppe mit bis zu 15 C-Atomen oder eine lineare oder verzweigte Alkylgruppe mit bis zu 15 C-Atomen stehen, wobei die Reste R und R2 so gewählt sind, dass das Keton der allgemeinen Formel (I) ein Duftstoffspeicher für Duftketone ausgewählt aus Buccoxim, iso-Jasmon, Methyl-beta-naphthylketon, Moschusindanon, Tonalid/Musk plus, alpha-Damascon, beta-Damascon, delta-Damascon, gamma-Damascon, Damascenon, Damarose, Methyldihydrojasmonat, Menthon, Carvon, Kampfer, Fenchon, alpha-Ionon, beta-Ionon, gamma-Methyl genannt Ionon, Fleuramon, Dihydrojasmon, cis-Jasmon, Methylcedrenylketon oder Methylcedrylon, Acetophenon, Methylacetophenon, para-Methoxyacetophenon, Methyl-beta-naphtylketon, Benzylaceton, Benzophenon, para-Hydroxyphenylbutanon, Sellerie-Keton oder Livescone, 6-Isopropyldecahydro-2-naphton, Dimethyloctenon, Frescomenthe, 4-(1-Ethoxyvinyl)-3,3,5,5-tetramethylcyclohexanon, Methylheptenon, 2-(2-(4-Methyl-3-cyclohexen-1-yl)propyl)cyclopentanon, 1-(p-Menthen-6(2)-yl)-1-propanon, 4-(4-Hydroxy-3-methoxyphenyl)-2-butanon, 2-Acetyl-3,3-dimethylnorbornan, 6,7-Dihydro-1,1,2,3,3-pentamethyl-4(5H)-indanon, 4-Damascol, Dulcinyl oderCassion, Gelson, Hexalon, Isocyclemon E, Methylcyclocitron, Methyllavendelketon, Orivon, para-tertiärem Butylcyclohexanon, Verdon, Delphon, Muscon, Neobutenon, Plicaton, Velouton, 2,4,4,7-Tetramethyl-oct-6-en-3-on, Tetrameran oder Mischungen davon ist.

2. Keton nach Anspruch 1, **dadurch gekennzeichnet, dass** der Substituent R2 für eine lineare oder verzweigte Alkylgruppe mit bis zu 6 C-Atomen, vorzugsweise bis zu 3 C-Atomen steht, insbesondere ein Methylrest ist.

3. Keton nach Anspruch 1 oder 2, **dadurch gekennzeichnet, dass** die Substituenten R3, R4, R5, R6 und R7 für Wasserstoff stehen.

4. Keton nach einem der Ansprüche 1 bis 2, entsprechend der Formel (II), (IV) oder Formel (V) 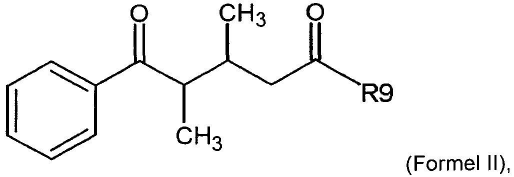 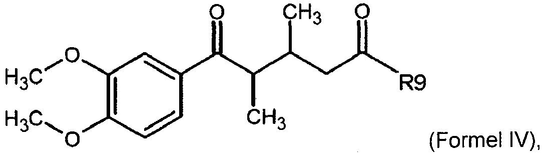 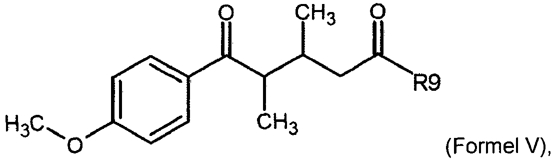 wobei der Rest R9 für einen Kohlenwasserstoffrest mit wenigstens 5 C-Atomen steht, der insbesondere einen cyclischen Kohlenwasserstoffrest umfasst.

5. Keton nach einem der Ansprüche 1, 2 und 4, entsprechend einer der folgenden Formeln (VI) bis (X), (XVI) bis (XXV)

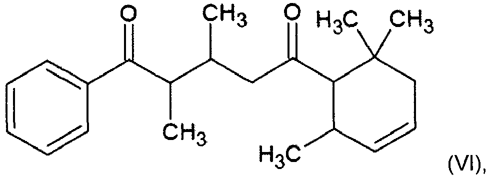

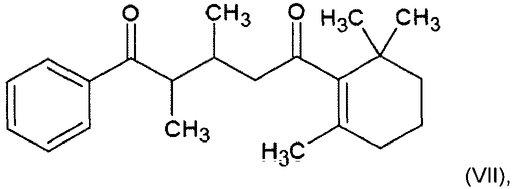

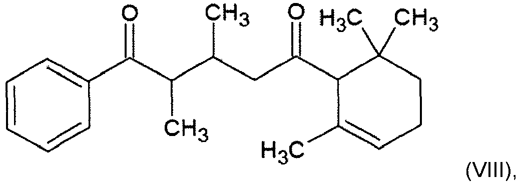

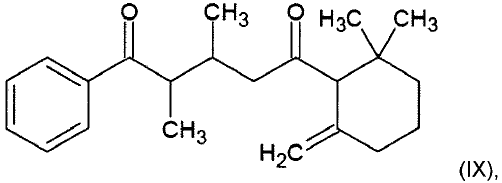

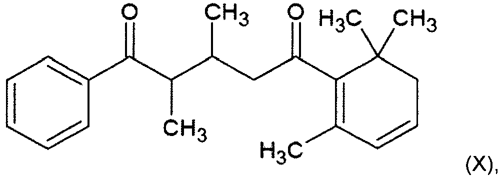

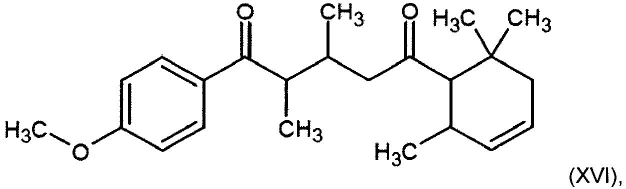

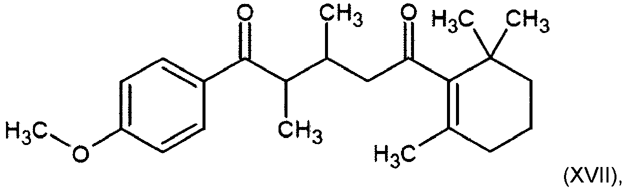

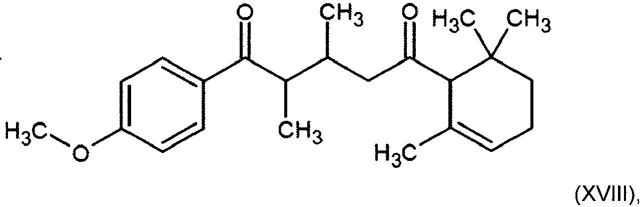

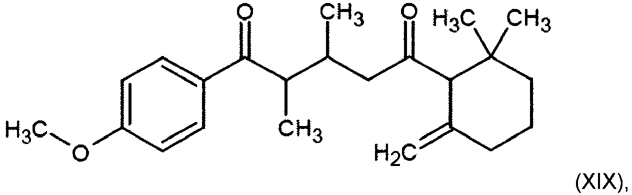

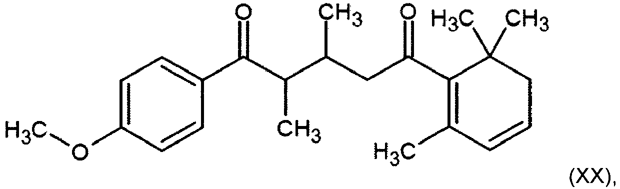

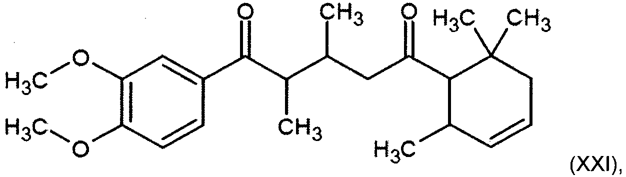

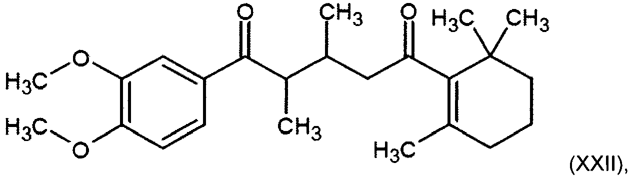

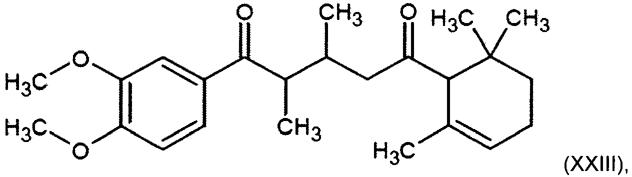

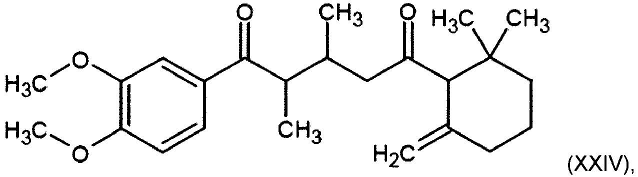

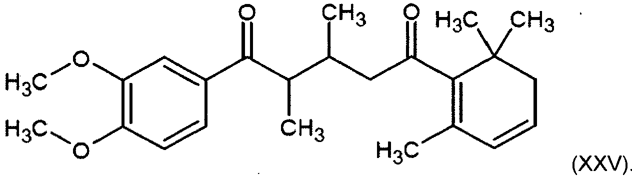

6. Wasch- oder Reinigungsmittel, enthaltend mindestens ein Keton nach einem der Ansprüche 1 bis 5, wobei das genannte Keton vorzugsweise in Mengen zwischen 0,0001 und 5 Gew.-%, vorteilhafterweise zwischen 0,001 und 4 Gew.-%, weiter vorteilhaft zwischen 0,01 und 3 Gew.-%, insbesondere zwischen 0,1 und 2 Gew.-%, jeweils bezogen auf das gesamte Mittel, enthalten ist.

7. Wasch- oder Reinigungsmittel nach Anspruch 6, **dadurch gekennzeichnet, dass** es mindestens ein Tensid ausgewählt aus der Gruppe bestehend aus anionischen, kationischen, nichtionischen, zwitterionischen, amphoteren Tensiden oder Mischungen daraus enthält.

8. Wasch- oder Reinigungsmittel nach einem der Ansprüche 6 oder 7, **dadurch gekennzeichnet, dass** es in fester oder flüssiger Form vorliegt.

9. Luftpflegemittel, enthaltend mindestens ein Keton nach einem der Ansprüche 1 bis 5, wobei das genannte Keton vorzugsweise in Mengen zwischen 0,0001 und 50 Gew.-%, vorteilhafterweise zwischen 0,001 und 5 Gew.-%, weiter vorteilhaft zwischen 0,01 und 3 Gew.-%, insbesondere zwischen 0,1 und 2 Gew.-%, jeweils bezogen auf das gesamte Mittel, enthalten ist.

10. Verfahren zur lang anhaltenden Beduftung von Oberflächen, **dadurch gekennzeichnet, dass** ein Keton nach einem der Ansprüche 1 bis 5 oder ein Mittel nach Anspruch 8 auf die zu beduftende Oberfläche aufgebracht wird und die genannte Oberfläche anschließend einer elektromagnetischen Strahlung umfassend die Wellenlängen von 200 bis 400 nm ausgesetzt wird.

## Claims

1. A ketone of the general formula (I) 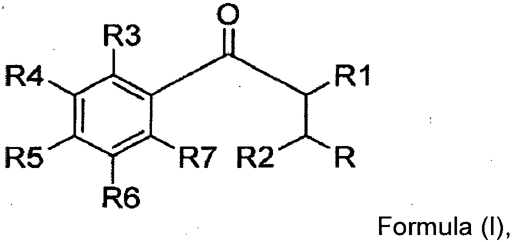 where
R denotes a hydrocarbon group that comprises at least one C=O group,
R1 denotes a linear or branched alkyl group having up to 6 carbon atoms,
R2, R3, R4, R5, R6, and R7 mutually independently denote hydrogen, a halogen atom, NO₂, a linear or branched alkoxy group having up to 15 carbon atoms, or a linear or branched alkyl group having up to 15 carbon atoms, the residues R and R2 being selected so that the ketone of the general formula (I) is a pro-fragrance for scent ketones selected from buccoxime, isojasmone, methyl beta-naphthyl ketone, musk indanone, Tonalid/Musk plus, alpha-damascone, beta-damascone, delta-damascone, gamma-damascone, damascenone, damarose, methyldihydrojasmonate, menthone, carvone, camphor, fenchone, alpha-ionone, beta-ionone, gamma-methyl (so-called) ionone, fleuramone, dihydrojasmone, cis-jasmone, methyl cedrenyl ketone or methyl cedrylone, acetophenone, methylacetophenone, para-methoxyacetophenone, methyl beta-naphthyl ketone, benzylacetone, benzophenone, para-hydroxyphenylbutanone, celery ketone or livescone, 6-isopropyldecahydro-2-naphthone, dimethyloctenone, frescomenthe, 4-(1-ethoxyvinyl)-3,3,5,5-tetramethylcyclohexanone, methylheptenone, 2-(2-(4-methyl-3-cyclohexen-1-yl)propyl)cyclopentanone, 1-(p-menthen-6(2)yl)-1-propanone, 4-(4-hydroxy-3-methoxyphenyl)-2-butanone, 2-acetyl-3,3-dimethylnorbornane, 6,7-dihydro-1,1,2,3,3-pentamethyl-4(5H)indanone, 4-damascol, dulcinyl or cassione, gelsone, hexalone, isocyclemone E, methylcyclocitrone, methyl lavender ketone, orivone, para-tert- butylcyclohexanone, verdone, delphone, muscone, neobutenone, plicatone, veloutone, 2,4,4,7-tetramethyloct-6-en-3-one, tetrameran, or mixtures thereof.

2. The ketone according to Claim 1, **characterised in that** the substituent R2 denotes a linear or branched alkyl group having up to 6 carbon atoms, by preference up to 3 carbon atoms, in particular is a methyl residue.

3. The ketone according to Claim 1 or 2, **characterised in that** the substituents R3, R4, R5, R6, and R7 denote hydrogen.

4. The ketone according to one of Claims 1 to 2, corresponding to formula (II), (IV), or formula (V) 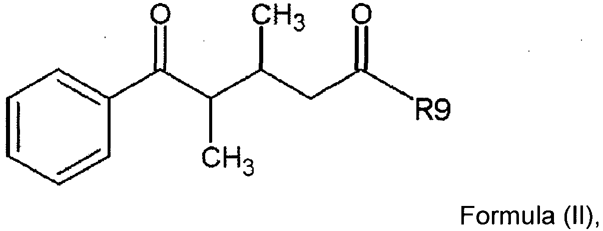 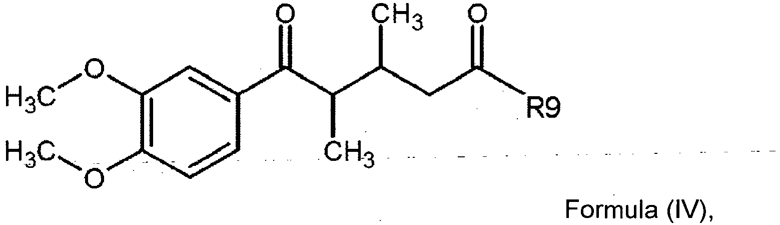 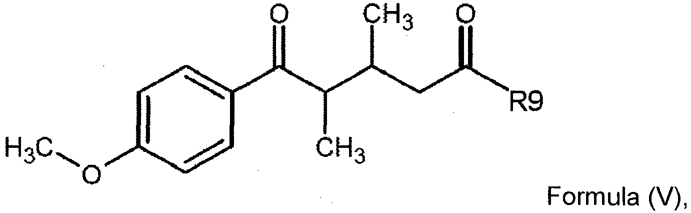 where the residue R9 denotes a hydrocarbon residue having at least 5 carbon atoms, which in particular comprises a cyclic hydrocarbon residue.

5. The ketone according to one of Claims 1, 2, and 4, corresponding to one of the following formulas (VI) to (X), (XVI) to (XXV)

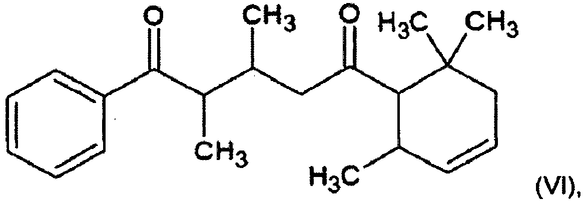

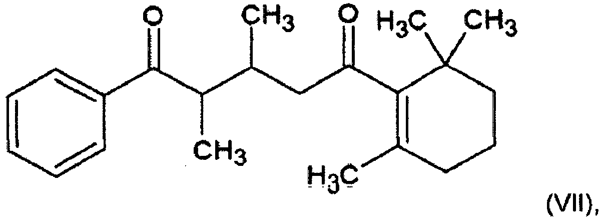

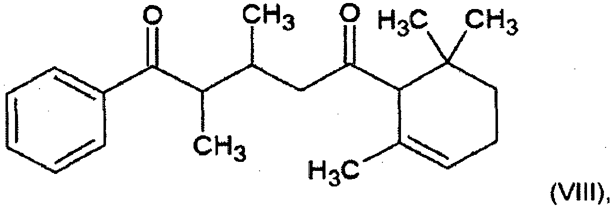

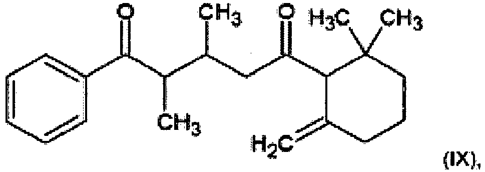

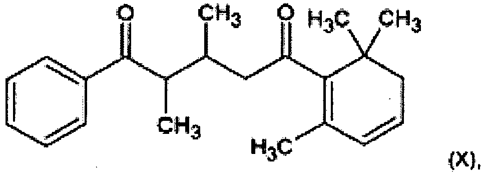

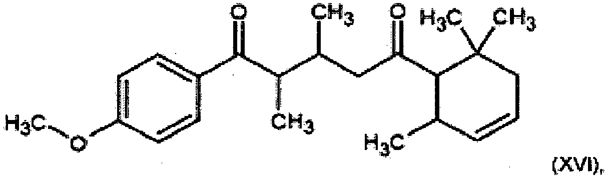

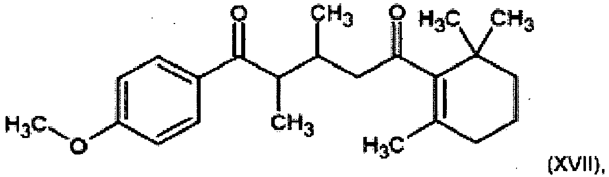

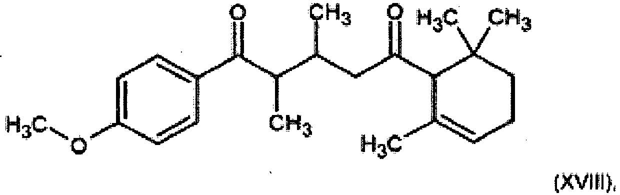

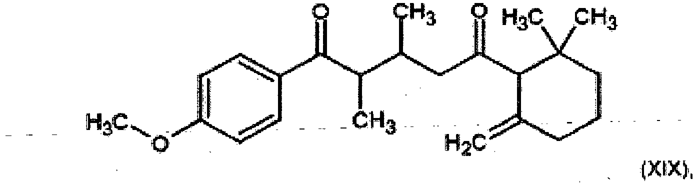

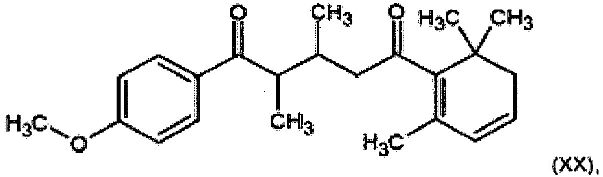

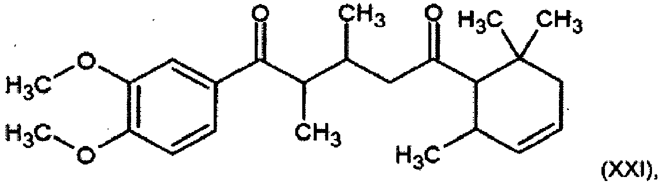

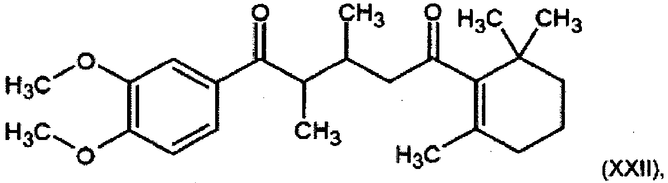

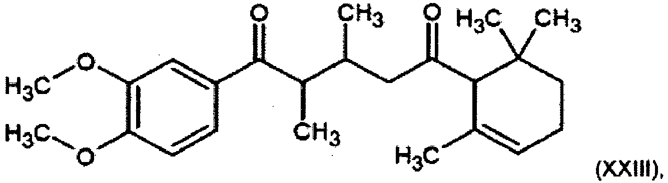

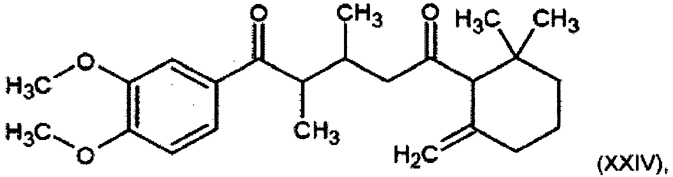

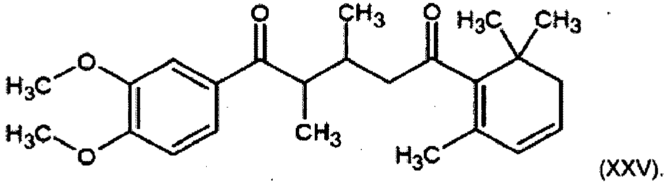

6. A washing or cleaning agent containing at least one ketone according to one of Claims 1 to 5, said ketone being contained by preference in quantities between 0.0001 and 5 wt%, advantageously between 0.001 and 4 wt%, more advantageously between 0.01 and 3 wt%, in particular between 0.1 and 2 wt%, based in each case on the total agent.

7. The washing or cleaning agent according to Claim 6, **characterised in that** it contains at least one surfactant selected from the group consisting of anionic, cationic, nonionic, zwitterionic, amphoteric surfactants or mixtures thereof.

8. The washing or cleaning agent according to one of Claims 6 or 7, **characterised in that** it is present in solid or liquid form.

9. An air freshening agent containing at least one ketone according to one of Claims 1 to 5, said ketone being contained by preference in quantities between 0.0001 and 50 wt%, advantageously between 0.001 and 5 wt%, more advantageously between 0.01 and 3 wt%, in particular between 0.1 and 2 wt%, based in each case on the total agent.

10. A method for long-lasting scenting of surfaces, **characterised in that** a ketone according to one of Claims 1 to 5 or an agent according to Claim 8 is applied onto the surface to be scented, and said surface is then exposed to an electromagnetic radiation comprising the wavelengths from 200 to 400 nm.

## Revendications

1. Cétone de la Formule générale (I), 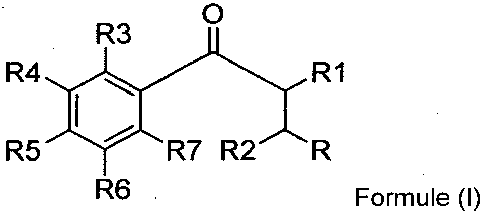 dans laquelle
R représente un résidu hydrocarbure qui comprend au moins un groupe C=O,
R1 représente un groupe alkyle linéaire ou ramifié comprenant jusqu'à 6 atomes de C,
R2, R3, R4, R5, R6 et R7 représentent indépendamment les uns des autres l'hydrogène, un atome d'halogène, NO₂, un groupe alcoxy linéaire ou ramifié comprenant jusqu'à 15 atomes de C ou un groupe alkyle linéaire ou ramifié comprenant jusqu'à 15 atomes de C, dans laquelle les résidus R et R2 sont sélectionnés de sorte que la cétone de la Formule générale (I) soit une matière accumulatrice de parfum pour des cétones parfumées sélectionnées parmi le buccoxime, l'isojasmone, la méthyl-bêta-naphtylcétone, l'indanone de musc, le tonalide/musc plus, l'alpha-damascone, la bêta-damascone, la delta-damascone, la gamma-damascone, la damascénone, le damarose, le dihydrojasmonate de méthyle, la menthone, la carvone, le camphre, la fenchone, l'alpha-ionone, la bêta-ionone, ladite gamma-méthylionone, la fleuramone, la dihydrojasmone, la cis-jasmone, la méthylcédrénylcétone ou la méthylcédrylone, l'acétophénone, la méthylacétophénone, la para-méthoxyacétophénone, la méthyl-bêta-naphtylcétone, la benzylacétone, la benzophénone, la para-hydroxyphénylbutanone, la cétone de céleri ou la livescone, la 6-isopropyldécahydro-2-naphtone, la diméthylocténone, la frescomenthe, la 4-(1-éthoxyvinyl)-3,3,5,5-tétraméthylcyclohexanone, la méthylhepténone, la 2-(2-(4-méthyl-3-cyclohexén-1-yl)propyl)cyclopentanone, la 1-(p-menthén-6(2)-yl)-1-propanone, la 4-(4-hydroxy-3-méthoxyphényl)-2-butanone, le 2-acétyl-3,3-diméthylnorbornane, la 6,7-dihydro-1,1,2,3,3-pentaméthyl-4(5H)-indanone, le 4-damascol, le dulcinyle ou la cassione, la gelsone, l'hexalone, l'isocyclémone E, la méthylcyclocitrone, la méthyl-lavande-cétone, l'orivone, la para-tert-butylcyclohexanone, la verdone, la delphone, la muscone, la néobuténone, la plicatone, la veloutone, la 2,4,4,7-tétraméthyl-oct-6-én-3-one, le tétramérane ou des mélanges de ceux-ci.

2. Cétone selon la revendication 1, **caractérisée en ce que** le substituant R2 représente un groupe alkyle linéaire ou ramifié comprenant jusqu'à 6 atomes de C, de préférence jusqu'à 3 atomes de C, est en particulier un résidu méthyle.

3. Cétone selon la revendication 1 ou 2, **caractérisée en ce que** les substituants R3, R4, R5, R6 et R7 représentent l'hydrogène.

4. Cétone selon l'une des revendications 1 à 2, correspondant à la Formule (II), (IV) ou à la Formule (V) 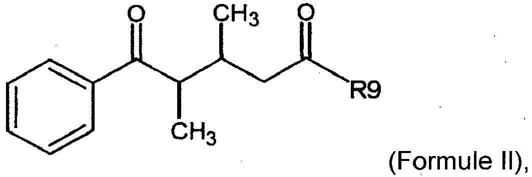 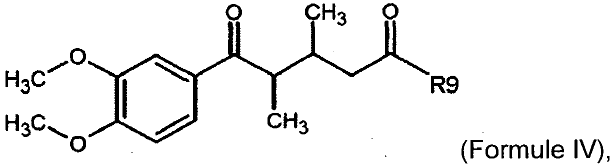 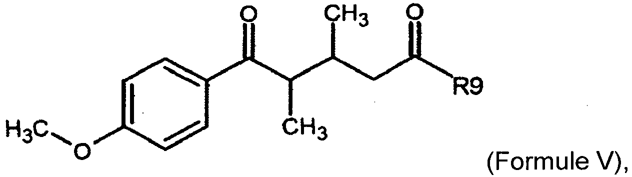 dans laquelle le résidu R9 représente un résidu hydrocarbure comprenant au moins 5 atomes de C, lequel comprend en particulier un résidu hydrocarbure cyclique.

5. Cétone selon l'une des revendications 1, 2 et 4, correspondant à l'une des Formules (VI) à (X), (XVI) à (XXV) suivantes

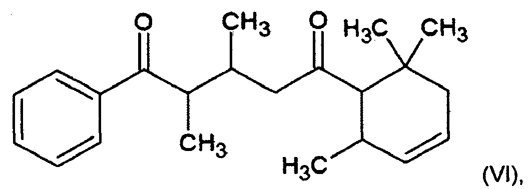

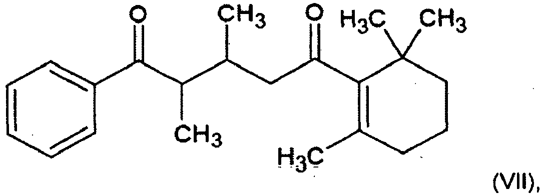

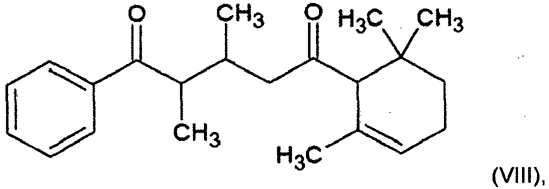

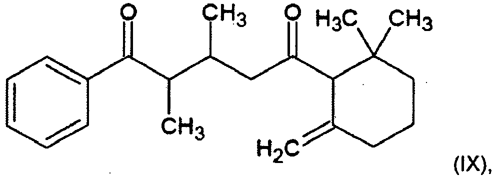

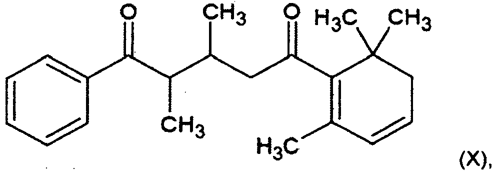

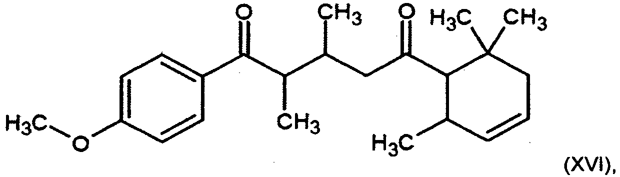

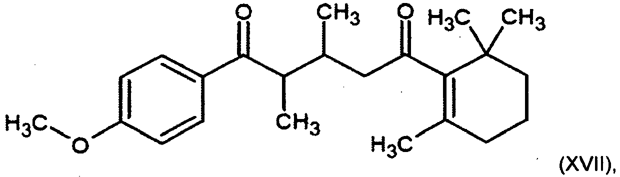

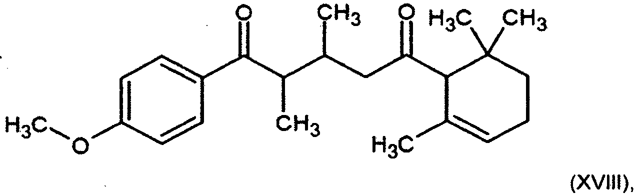

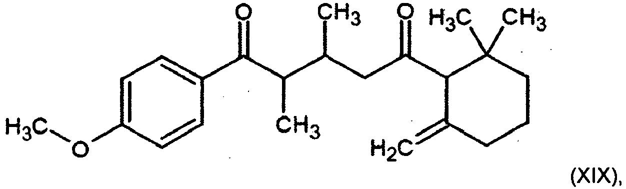

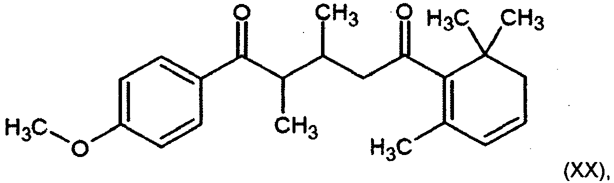

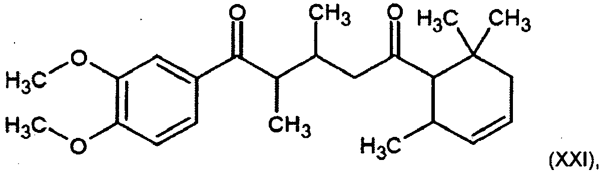

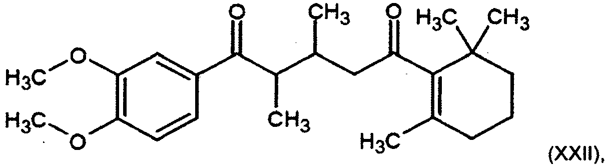

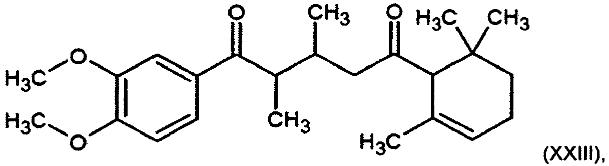

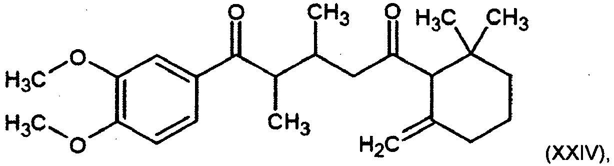

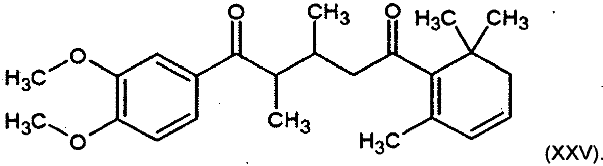

6. Agent de lavage ou de nettoyage, contenant au moins une cétone selon l'une des revendications 1 à 5, dans lequel ladite cétone est contenue de préférence dans des quantités comprises entre 0,0001 et 5 % en poids, de manière avantageuse entre 0,001 et 4 % en poids, de manière encore plus avantageuse entre 0,01 et 3 % en poids, en particulier entre 0,1 et 2 % en poids, rapporté respectivement à l'agent total.

7. Agent de lavage ou de nettoyage selon la revendication 6, **caractérisé en ce qu'**il contient au moins un agent tensioactif sélectionné dans le groupe constitué d'agents tensioactifs anioniques, cationiques, non ioniques, zwitterioniques, amphotères, ou de mélanges de ceux-ci.

8. Agent de lavage ou de nettoyage selon l'une des revendications 6 ou 7, **caractérisé en ce qu'**il se présente sous forme solide ou liquide.

9. Agent désodorisant, contenant au moins une cétone selon l'une des revendications 1 à 5, dans lequel ladite cétone est contenue de préférence dans des quantités comprises entre 0,0001 et 50 % en poids, de manière avantageuse entre 0,001 et 5 % en poids, de manière encore plus avantageuse entre 0,01 et 3 % en poids, en particulier entre 0,1 et 2 % en poids, rapporté respectivement à l'agent total.

10. Procédé pour le parfumage longue durée de surfaces, **caractérisé en ce qu'**une cétone selon l'une des revendications 1 à 5 ou un agent selon la revendication 8 est appliqué(e) sur la surface à parfumer et ladite surface est ensuite exposée à un rayonnement électromagnétique comprenant les longueurs d'onde de 200 à 400 nm.
